# EUROPEAN PATENT APPLICATION

(11) **EP 4 732 822 A1**
(43) Date of publication of application: **29.04.2026**
(21) Application number: 25208372.0
(22) Date of filing: 13.10.2025
(51) Int. Cl.: A61H 33/00

(54) **A NOZZLE ASSEMBLY**

(30) Priority: 22.10.2024 GB 202415578
(71) Applicant: Superior Wellness Ltd, Chesterfield, Derbyshire S41 9RT (GB)
(72) Inventor: CARLIN, Rob, Chesterfield, S41 9RT (GB)
(74) Representative: Sugden, Mark William

(57) **Abstract**

A nozzle assembly is provided for a hot tub or hydrotherapy system. The nozzle assembly includes: a nozzle body with internal cavity; an inlet port upstream of the cavity; an outlet port downstream of the cavity to define a flow path between the inlet port and the outlet port. The nozzle assembly also includes a light emitting element configured to emit red light.

## Description

### FIELD OF THE INVENTION

The present teachings relate to a nozzle assembly for a hot tub or hydrotherapy system, and to a hot tub or hydrotherapy system.

### BACKGROUND OF THE INVENTION

Hot tubs or hydrotherapy systems are used for relaxation, personal enjoyment or physical therapy. Both hot tubs and hydrotherapy systems deliver pressurised water to a basin or receptacle of the hot tub/system that receives the user's body to massage the skin and joints through a kneading sensation.

The delivery of the pressurised water aims to enhance the user's overall wellness experience, either by relaxing the user or providing rehabilitation to help address inflammation or aches and pains. The pressurised water is typically delivered by nozzles that are mounted to the sidewall or base wall of the hot tub/hydrotherapy system.

To increase the relaxing experience of rejuvenation of the user, the position and strength of flow from the nozzles can be adjusted depending on the user and the location of their pain. The rate of pressurised water delivery can be preprogrammed into the tub or hydrotherapy system or is controllable by the user.

It is desirable to improve the experience of the user in a hot tub or hydrotherapy system, either for relaxation or rejuvenation purposes.

### SUMMARY OF THE INVENTION

According to a first aspect there is provided a nozzle assembly for a hot tub or hydrotherapy system, the nozzle assembly comprising a nozzle body comprising an internal cavity for receiving liquid therein, an inlet port upstream of the cavity for conveying liquid into the cavity, an outlet port configured to dispense liquid and arranged downstream of the cavity to define a flow path between the inlet port and the outlet port, and a light emitting element supported by the nozzle, wherein the light emitting element is configured to emit red light.

By providing red light, the nozzle assembly is able to provide improved health benefits to the user of the hot tub or hydrotherapy system. Red light provides general health benefits, such as improving circulation and reducing inflammation.

Optionally, the light emitting element is at least partially, for example entirely, arranged in the cavity.

The nozzle assembly therefore is compact and self-contained. The hot tub or hydrotherapy system does not need to be further adapted to receive the nozzle assembly. The light emitting element is also protected from external damage.

Optionally, the light emitting element is arranged within the cavity such that the red light is emitted through the outlet port.

In this arrangement, the outlet has a dual function of providing a light emitting element and delivering liquid from the outlet. This provides a more compact nozzle assembly that does not reduce the flow rate of the nozzle whilst incorporate red lights.

Optionally, the light emitting element is aligned with the outlet port.

This helps to increase the amount of red light emitted through each outlet port.

Optionally, the red light has a wavelength in the range 620nm to 750nm, for example in the range 630nm to 700nm.

By providing red light, the nozzle assembly is able to provide improved health benefits to the user of the hot tub or hydrotherapy system. Red light provides general health benefits, such as improving circulation and reducing inflammation.

Optionally, the outlet port comprises a plurality of first outlet ports having a corresponding light emitting element aligned therewith, and at least one second outlet port not aligned with a light emitting element.

In some embodiments, the at least one second outlet port is at least partially aligned with the inlet port.

In some embodiments, the plurality of first outlet ports surround the at least one second outlet port.

Optionally, the light emitting element is arranged within the cavity to be spaced apart from the outlet port.

In this arrangement, the nozzle assembly can provide an unobstructed flow of liquid through the nozzle assembly. The performance and the rate of liquid delivered through the nozzle is therefore not affected by the presence of the light emitting element.

Optionally, the flow nozzle further comprises a plurality of light emitting elements that are arranged to surround the inlet port.

In this arrangement, the light emitting elements do not obstruct the flow of liquid through the cavity.

Optionally, the plurality of light emitting elements are arranged in a circular arrangement.

Optionally, the flow nozzle comprises a plurality of outlet ports.

The plurality of outlet ports enables the nozzle assembly to maximise the amount of liquid delivered through the nozzle.

Optionally, the plurality of outlet ports are arranged in a circular arrangement.

In this arrangement, an even distribution of liquid is delivered from the nozzle assembly. This is particularly advantageous for providing an even distribution of pressurised liquid from the nozzle. Pressurised liquid has health benefits such as massaging and improving circulation.

Optionally, the nozzle assembly comprises a plurality of light emitting elements and a corresponding plurality of outlet ports, each light emitting element aligned with a corresponding outlet port.

As such, the intensity of light emitted from each light emitting element can be controlled through each outlet. The nozzle assembly can therefore maximise the amount of light that it emits.

Optionally, the light emitting element is electrically isolated from the flow path.

The light emitting element is therefore protected from the flow of liquid through the nozzle assembly.

Optionally, the outlet port is formed on an outlet body, and wherein said outlet body is removably mounted to the nozzle body.

The nozzle assembly can therefore be easily assembled and disassembled. This can help retrofit the nozzle assembly onto existing hot tubs or hydrotherapy systems, or for repairing specific parts of the nozzle assembly, for example the light emitting elements.

Optionally, the outlet body comprises a mounting arrangement to mount said outlet body to the nozzle body.

Optionally, the mounting arrangement comprises a threaded region on an external surface of the outlet body configured to mate with a corresponding threaded surface on the nozzle body.

Optionally, a plurality of light emitting elements are provided. In some embodiments, the plurality of light emitting elements are mounted to a lighting body. The lighting body may be provided as a removable sub-assembly.

Optionally, the second part comprises an opening that forms part of the flow path between the inlet port and outlet port.

Optionally, the second part has a diameter of 7 inches.

Optionally, the flow nozzle further comprises a housing comprising a mounting arrangement to mount the nozzle assembly to a hot tub or hydrotherapy system.

The nozzle assembly can therefore be retrofitted onto existing hot tubs or hydrotherapy systems

Optionally, the mounting arrangement comprises a threaded region on an external surface of the nozzle body.

According to a second aspect, there is provided a hot tub and/or a hydrotherapy system comprising the nozzle assembly of the first aspect.

### BRIEF DESCRIPTION OF THE DRAWINGS

Embodiments of the invention will now be described with reference to the accompanying drawings, in which:
Figure 1A shows an exemplary hot tub;
Figure 1B shows a schematic drawing of the systems in an exemplary hot tub;
Figure 2 shows an exemplary nozzle assembly arrangement;
Figure 3 shows a cross-sectional view of an exemplary nozzle assembly;
Figure 4 shows an expanded view of an exemplary nozzle assembly;
Figure 5 shows a light emitting element in greater detail;
Figure 6A shows an exemplary schematic drawing of a nozzle assembly;
   and
Figure 6B shows another exemplary schematic drawing of a nozzle assembly.

### DETAILED DESCRIPTION OF EMBODIMENT(S)

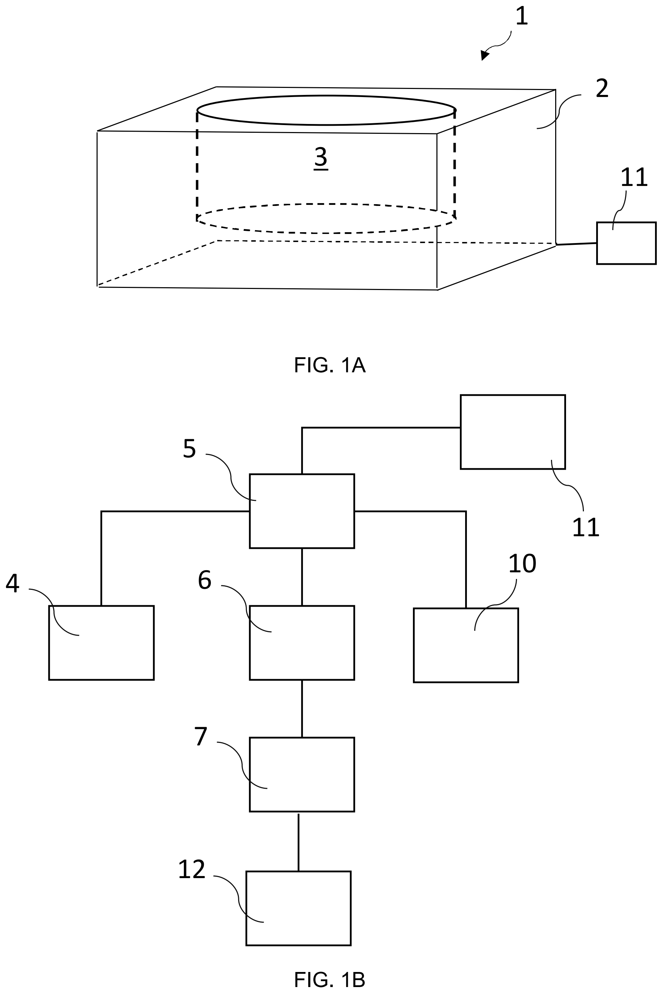

Figure 1A shows an exemplary hot tub 1 or a hydrotherapy system. While the illustrated example of Figure 1 is in reference to a hot tub 1, it will be appreciated that the nozzle assembly described herein may be equally used in a hydrotherapy system, a spa, swim spa, whirlpool bathtub or any other water treatment system.

The hot tub 1 includes a housing or body 2. The hot tub 1 includes a shell 3 mounted within the housing 2. The shell 3 forms a basin or receptacle and is capable of receiving a volume of water and is large enough to accommodate one or more users therein. Although not illustrated, the shell 3 may include one or more seats for one or more users to be seated thereon.

The hot tub 1 includes a heating unit 4, a water circulation system 6 and a filtration system 10, shown schematically in Figure 1B. These systems are typically integrated with the hot tub 1 and are arranged to control the operation of the hot tub 1.

The heating unit 4 is configured to maintain the temperature of the liquid provided in the basin or receptacle at a desired temperature. The heating unit 4 may maintain the temperature in the basin or receptacle in any suitable manner readily known, such as using a heating element and/or utilising thermostat control (not shown).

The water circulation system 6 controls the flow of water into the basin or receptacle. The water circulation system 6 includes a pump 7, and a series of nozzles 12. The pump 7 provides pressurised water to the nozzle assemblies by controlling the water flow and pressure, thereby acting as a generator. The pressurised water is delivered to the basin or receptacle through one or more nozzles 12. While only one nozzle 12 is shown schematically in Figure 1B, it will be understood that any number and arrangement of nozzles 12 may be provided in the hot tub 1.

The filtration system 10 is provided to remove impurities from the water before it is delivered into the basin or receptacle. The filtration system 10 may be provided in any suitable form, such as ceramic filters, cartridge filters or sand filters.

The hot tub 1 may be connected to an external source of electrical power 11 for powering the circulation system 6, the pump 7 and or the filtration system 10. Alternatively, the hot tub 1 may include the power source 11, for example in the form of a battery.

The heating unit 4, water circulation system 6, and the filtration system 10 may be electronically connected to a control panel 5. The control panel 5 is also powered by the power source 11. The control panel 5 is arranged to control the operation of the heating unit 4, water circulation system 6 and the filtration system 10, and may also receive information from each system. The control panel 5 is preferably operable by the user and may be presented in any suitable accessible form, such as a touch screen integrated with the hot tub 1 or a separate device that is electronically connected to the hot tub 1. The control panel 5 is therefore operable by a user so that the temperature, water pressure from the flow nozzles and other parameters are easily customisable during a user's use of the hot tub 1.

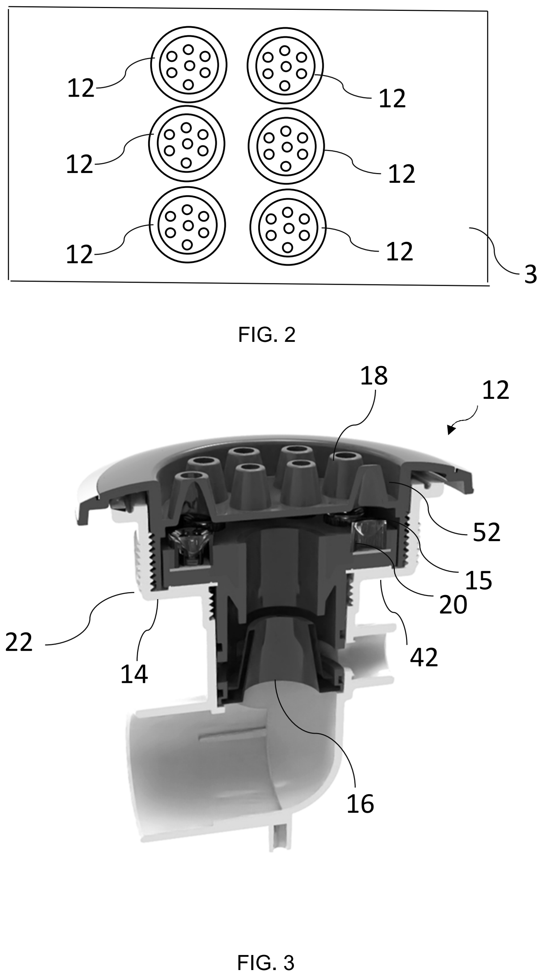

The nozzles 12 are positioned along the side walls and/or base of the shell 3 to provide a flow of water into the basin or receptacle. Figure 2 shows an exemplary arrangement of six nozzles 12 arranged on a wall of the shell 3. It will be understood that any number, arrangement or position of nozzles 12 may be provided in the hot tub 1.

Referring to Figure 3, a cross-sectional view of an exemplary nozzle or nozzle assembly 12 is illustrated. The nozzle assembly 12 is includes a nozzle body 14 defining an internal cavity 15 for receiving liquid, for example water, therein. The nozzle assembly 12 includes an inlet port 16, and an outlet port 18 configured to dispense liquid into the basin or receptacle. The inlet port 16 is arranged to receive water from a source (such as the pump 6 or piping connected to the pump 6). The pump 6 maybe upstream of the inlet 16 within the hot tub 1 or be provided outside the hot tub 1 as an external structure. The inlet port 16 is arranged upstream of the cavity 15 for conveying liquid into the cavity 15. The outlet port 18 is arranged downstream of the cavity 15 to define a flow path from the inlet port 16, through the cavity 15, and to the outlet port 18.

In the illustrated embodiment, the nozzle assembly 12 includes a plurality, or an array, or outlet ports 18. By providing several outlet ports 18, the nozzle assembly 12 can not only maximise the amount of water delivered from the nozzle assembly 12, but also maintain the pressure of the water that is delivered to the inlet port 16. The plurality of outlets 18 also provides an even distribution of liquid from the nozzle 12, which has an improved massaging effect. Although the nozzle 12 is shown to have several exemplary outlet ports 18 in Figure 3, it will be understood that the nozzle body 14 may have any number of outlet ports 18, or alternatively, may only have one outlet port 18. Each outlet port 18 is provided having a generally frustoconical shape, but it will be appreciated that any suitable outlet port 18 may be used in alternative embodiments.

The nozzle assembly 12 has a mounting arrangement 22 that is used to mount the nozzle assembly 12 to the hot tub 1. In particular, the mounting arrangement 22 is provided to mount the nozzle assembly 12 to the shell 3. In this example, the mounting arrangement 22 is provided on the nozzle body 14. In the illustrated example the mounting arrangement 22 is provided in the form of an external thread on an exterior surface of the nozzle body 12. The external thread is configured to attach to a corresponding threaded aperture (not shown) in the shell 3. This enables the nozzle assembly 12 to be easily installed and removed from the shell 3 of the hot tub 1 for installation, repair or maintenance. In other embodiments, the mounting arrangement 22 may be provided in any suitable form, for example a fastening arrangement, such as bolts, clamps or fasteners, and may be arranged on any suitable part of the nozzle assembly 12. It will also be understood that the mounting arrangement 22 may be provided at any suitable on the nozzle assembly 12. Once the nozzle assembly 12 is installed into the hot tub 1, or more specifically the shell 3, the outlet port(s) 18 are arranged to be in fluid communication with the basin or receptacle so that liquid from the pump 6 is delivered directly into the basin or receptacle of the shell 3.

The nozzle assembly 12 includes a lighting assembly. The lighting assembly includes a light emitting element 20. The light emitting element 20 may be arranged within the cavity 15. In other embodiments, the light emitting element 20 may be arranged at any suitable location on the nozzle assembly 12. As is illustrated, the light emitting element 20 maybe spaced apart from the outlet port 18. This minimises the risk of the light emitting element 20 obstructing the flow path of the liquid through the cavity 15. Therefore, the outlet 18 can still perform the maximum function of delivering fluid to the outlets 18.

In this example, the light emitting element 20 is supported on the nozzle assembly 12. In the illustrated embodiment, a plurality of light emitting elements 20 are provided. In alternative arrangements, it will be understood that any suitable number of light emitting elements 20 may be provided. The light emitting element may be a light emitting diode (LED), or any other suitable light. The or each light emitting element 20 is configured to emit red light. The red light emitted from the light emitting element 20 may be any suitable infrared light, or any red light. The red light emitted by the or each light emitting element 20 may have a wavelength in the range 620 nanometres to 750 nanometres. In some embodiments, the red light emitted from the or each light emitting element 20 may be in the range 630 nanometres to 700 nanometres. In the arrangement shown, the light emitting element(s) are arranged within the cavity 15 such that the red light is emitted through the outlet port(s) 18.

In some embodiments, the or each light emitting element 20 may be partially or fully aligned with a corresponding outlet port 18. In the illustrated example, the nozzle assembly includes a plurality of light emitting elements 20. Each light emitting element 20 may be arranged in the nozzle assembly 12 to be aligned with a corresponding outlet port 18. Each outlet port that is aligned with a corresponding light emitting element 20 may be considered to be a first outlet port 18a. In some embodiments, the nozzle assembly may also include one or more second outlet ports 18b that are not aligned with a light emitting element. In some embodiments, the or each second outlet port may be at least partially aligned with the inlet port 16. In the illustrated example, the plurality of first outlet ports 18a are arranged to surround the or each second outlet port 18b.

By providing red light through the nozzle assembly 12, the user of the hot tub 1 can not only enjoy improved rejuvenation and relaxation from the pressurised flow of liquid from the nozzle assemblies 12, but also receive red light therapy from the light emitting elements 18. This is achieved without having to reduce the number of water nozzles (i.e. by replacing them with red lights) and without having to increase the number of separate lights and nozzles mounted to the shell 3. Red light exposure has several benefits on the skin and/or joints of a user, such as enhancing blood circulation, reducing inflammation, promoting faster would healing and improving skin conditions. Therefore, the user of the hot tub 1 can experience an improved rejuvenation and relaxation experience with the dual action of pressurised water and red light therapy from the nozzle assemblies 12. In other examples, one or more light emitting element 20 may be configured to emit visible, for example white, light to illuminate the flow nozzle 12. This can be used to replace dedicated lighting systems within the hot tub 1.

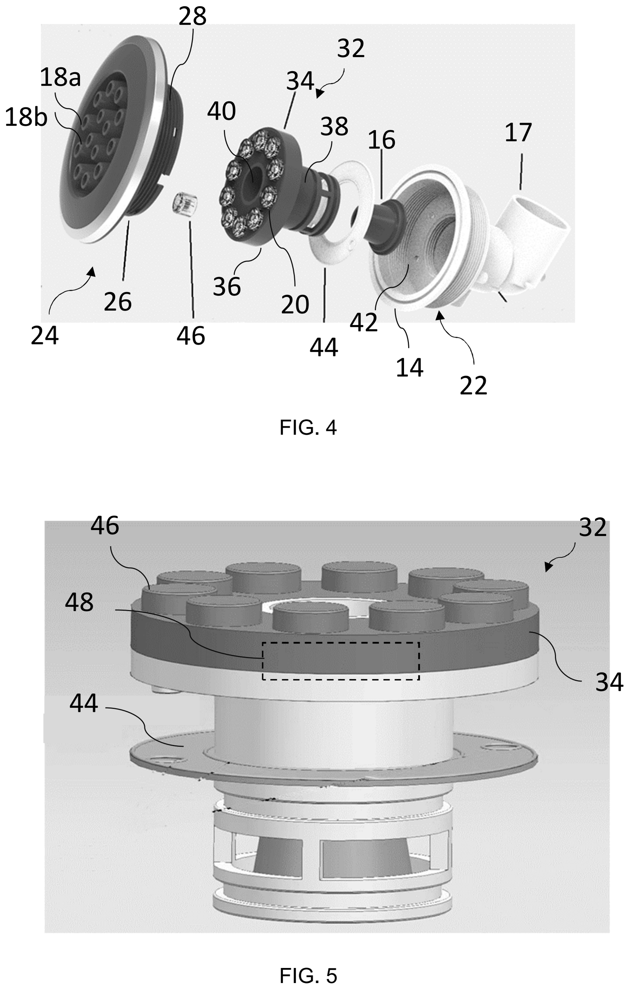

Referring now to Figures 4 and 5, the nozzle assembly 12 includes an outlet body 24 that is removably mounted to the nozzle body 14. A lighting arrangement 32 is arranged between the outlet body 24 and the nozzle body 14. An upstream end of the nozzle body 14 is connected to pipework 17. The pipework may be integrally formed with the nozzle body 14, or may be connectable to thereto. The pipework 17 is fluidly connected to the water circulation system 6 to receive water therefrom. The nozzle assembly 12 is therefore a compact arrangement as it is provided in three parts 14, 24, 32, that are arranged to be assembled together. It is therefore easy to assemble and disassemble the nozzle assembly 12 as it is provided as a self-contained unit. The nozzle assembly 12 can therefore be easily removed, for example, for repair, or retrofitted into existing hot tubs 1.

The nozzle assembly 12 includes an outlet body 24. The outlet body 24 includes the outlet port(s) 18. The outlet body 24 is removably mounted to the nozzle body 14. The outlet body 24 includes a mounting arrangement thereon to removably mount the outlet body 24 to the nozzle body 14. In the illustrated embodiment, a radially outer surface 26 includes a threaded surface 28 that is configured to mount onto a corresponding threaded internal surface 30 of the nozzle body 14. In other embodiments, it will be appreciated that any suitable mounting arrangement may be used to mount the outlet body 18 to the nozzle body, for example fasteners, such as bolts or clips.

As discussed above, the nozzle body 14 defines an internal cavity 15. The lighting arrangement 32 is arranged within the cavity 15. The lighting arrangement 32 is provided as a removable body or sub-assembly 34 of the nozzle assembly 12. The lighting body 34 has a cylindrical upper portion 36 with a stem 38 extending from the upper portion 36. The upper portion 36 and stem 38 may be formed as a unitary component or may be provided as two separate components fastened together by any suitable means, such as glue or by fasteners. The lighting body 34 has a through-hole or bore 40 extending therethrough. The bore 40 extends through both the upper portion 36 and the stem 38. The bore 40 forms a downstream end of the inlet port 16.

The lighting body 34 is seated on an internal shoulder 42 of the nozzle body 14. In the illustrated embodiments, a sealing member 44 is positioned between the lighting body 34 and the shoulder 42. In the arrangement shown, the sealing member 44 is provided as an annular ring, for example made of rubber

Referring now to Figure 5, the lighting arrangement 32 includes a light emitting element 20 supported on the lighting body 34. In this example, the light emitting element 20 is a LED light source covered by a transparent protective casing 46. The light emitting elements 20 are arranged on an upper surface of the lighting body 3 such that they are directed to the outlet port(s) 18. The light emitting elements 20 are electrically connected to a circuit 48 (shown schematically). The circuit 48 may be contained and protected within the lighting body 34. Therefore, the liquid passing through the nozzle assembly 12 does not interfere with any of the electrical components of the lighting arrangement 32. The circuit 48 may be powered by a self-contained power source (not shown), such as a battery. In other examples, the circuit 48 may be electrically connected to the power source 11 used to power other components of the hot tub 1, such as the pump 7.

The nozzle assembly 12 may include any number of light emitting elements 20. For example, nozzle assembly 12 may have several light emitting elements 20, or only one light emitting element 20, depending on the operation of the nozzle assembly 12. As described above, the lighting body 34 includes a bore 40 that defines a downstream end of the inlet port 16. The light emitting elements 20 are arranged in around the bore 40 (i.e. around the inlet port 16), for example in a circular arrangement. The light emitting elements 20 therefore surround the inlet port 16 and do not obstruct the flow path. The light emitting elements 20 are provided as individual LED lights that are individually encapsulated in a protective cover 21. However, it will be understood that alternative light arrangements may be used, such as multiple LED lights with one protective cover, or one continuous strip light.

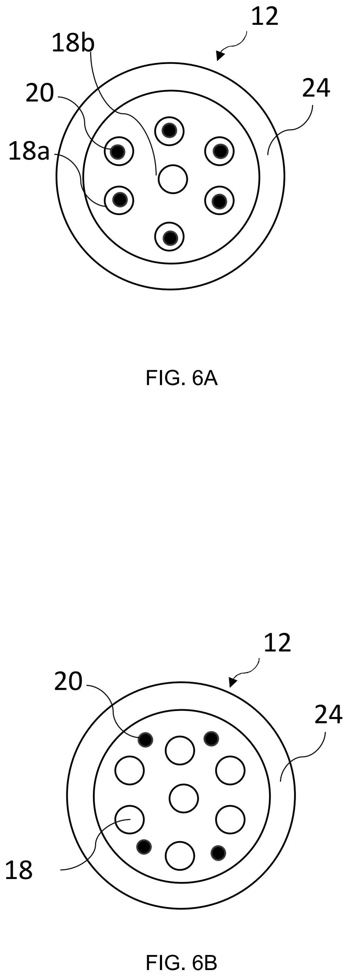

The lighting body 34 is arranged within the cavity 15 such that the light emitting elements 20 are positioned behind the outlet ports 18. This enables the red light from the light emitting elements to be emitted through the outlet ports 18. In the illustrated arrangement, the light emitting elements 20 are arranged adjacent to the outlets 18. In this arrangement, the light emitting elements 20 may be aligned with the outlets 18 to allow the maximum amount of light to be emitted through the outlet 18 (see Figure 6A). In other examples, the light emitting elements 20 may be arranged offset from the outlets 18 so only some light from the light emitting elements 20 is emitted through the outlets 18 (see Figure 6B).

Thus, in the nozzle assembly 12 of the present teachings, the outlet ports 18 therefore provide a dual function of providing pressurised water from the pump 7 through the outlets, as well as providing light from the light emitting elements 20. In both arrangements of Figures 6A and B, the light emitting elements 20 emit light without disrupting the flow of liquid from the outlets 18. The nozzle assembly 12 can therefore continue to deliver the optimum flowrate and volume of liquid through the nozzle 12 and into the basin or receptacle of the hot tub 1. The performance of the nozzle assembly 12 is therefore not hindered by the inclusion of the light emitting element 20.

Although the invention has been described above with reference to one or more preferred embodiments, it will be appreciated that various changes or modifications may be made without departing from the scope of the invention as defined in the appended claims.

## Claims

1. A nozzle assembly for a hot tub or hydrotherapy system, comprising:
a nozzle body comprising an internal cavity for receiving liquid therein;
an inlet port upstream of the cavity for conveying liquid into the cavity;
an outlet port for configured to dispense liquid, said outlet port arranged downstream of the cavity to define a flow path from the inlet, through the cavity to the outlet port; and
a light emitting element;
wherein the light emitting element is configured to emit red light.

2. The nozzle assembly according to claim 1, wherein the light emitting element is arranged within the cavity such that the red light is emitted through the outlet port.

3. The nozzle assembly according to claim 2, wherein the light emitting element is aligned with the outlet port.

4. The nozzle assembly according to any preceding claim, comprising a plurality of light emitting elements and a corresponding plurality of outlet ports, wherein each light emitting element is aligned with a corresponding outlet port.

5. The nozzle assembly according to claim 4, wherein the plurality of light emitting elements are arranged around the inlet port and/or wherein the plurality of light emitting elements are arranged in a circular arrangement.

6. The nozzle assembly according to any preceding claim, wherein the outlet port comprises a plurality of first outlet ports having a corresponding light emitting element aligned therewith, and at least one second outlet port not aligned with a light emitting element.

7. The nozzle assembly according to claim 6, wherein the at least one second outlet port is at least partially aligned with the inlet port.

8. The nozzle assembly according to claim 6 or claim 7, wherein the plurality of first outlet ports surround the at least one second outlet port.

9. The nozzle assembly according to any one of claims 6 to 8, wherein the at least one second outlet port is at least partially aligned with the inlet port.

10. The nozzle assembly according to any preceding claim, wherein the light emitting element is arranged within the cavity to be spaced apart from the outlet port.

11. The nozzle assembly according to any preceding claim, wherein the light emitting element is electrically isolated from the flow path.

12. The nozzle assembly according to any preceding claim, wherein the outlet port is formed on an outlet body, and wherein said outlet body is removably mounted to the nozzle body.

13. The nozzle assembly according to claim 12, wherein the outlet body comprises a mounting arrangement to mount said outlet body to the nozzle body, optionally wherein the mounting arrangement comprises a threaded region on an external surface of the outlet body configured to mate with a corresponding threaded surface on the nozzle body.

14. The nozzle assembly according to any preceding claim, comprising a plurality of light emitting elements mounted to a lighting body, and wherein the lighting body is provided as a removable sub-assembly, optionally wherein the lighting body comprises a central bore defining a downstream end of the inlet port.

15. A hot tub and/or hydrotherapy system comprising the nozzle assembly of any preceding claim.
